# EUROPEAN PATENT APPLICATION

(11) **EP 3 054 017 A1**
(43) Date of publication of application: **10.08.2016**
(21) Application number: 15153705.7
(22) Date of filing: 03.02.2015
(51) Int. Cl.: C12Q 1/68, C12N 15/113, A61K 31/7088, A61P 9/00

(54) **Circular RNA for the diagnosis and treatment of cardiovascular diseases**

(71) Applicant: Johann Wolfgang Goethe-Universität, Frankfurt am Main, 60323 Frankfurt am Main (DE)
(72) Inventor: Boeckel, Niels, 60385 Frankfurt (DE); Dimmeler, Stefanie, 60594 Frankfurt (DE); Zeiher, Andreas M., 60594 Frankfurt (DE); Jaé, Nicolas, 63540 Hanau (DE)
(74) Representative: Krauss, Jan

(57) **Abstract**

The present invention associates multiple circular (circRNA) with key functions of endothelial cells. The present invention provides circRNA transcripts that are correlated with cardiovascular diseases, in particular acute myocardial infarction. The circRNA of the invention therefore serve as biomarkers in the diagnosis of cardiovascular disorders. The invention provides the new nucleic acid molecules as well as diagnostic kits and compositions comprising the nucleic acids. Modulation of the expression of the circRNA of the invention further leads to endothelial cell sprouting, and therefore is applied as a treatment for cardiovascular diseases or pathological angiogenesis. The invention provides therapeutic agents modulating circRNA expression or function.

SEQUENCE LISTING

<110>  Goethe Universität Frankfurt am Main

<120>  CIRCULAR RNA FOR THE DIAGNOSIS OF CARDIOVASCULAR DISEASES

<130>  U30607EP

<160>  32

<170>  PatentIn version 3.5

<210>  1
<211>  80
<212>  RNA
<213>  Homo sapiens

<400>  1
auguuguuug cauugacuug gauacgaaca uguuauaugu aguucuauau gaagggaaag      60

aacggcuuau uccaggaugu                                                  80


<210>  2
<211>  80
<212>  RNA
<213>  Homo sapiens

<400>  2
caacacagaa ucguccucuu uggcaauguu uucuuggacg aauuuagcuu ggguuaaauc      60

auauaacauc ugcuuugaau                                                  80


<210>  3
<211>  80
<212>  RNA
<213>  Homo sapiens

<400>  3
caagcucacc aaacugaaga ugccuucuca gucaguugag uuuguacaau gacgacagaa      60

accugcuucg aauuagagag                                                  80


<210>  4
<211>  80
<212>  RNA
<213>  Homo sapiens

<400>  4
ugcacuauuc uuucccagga accauuggau aaggauaaag gcuugcccca gaacacacac      60

uauagagcag augcaguagc                                                  80


<210>  5
<211>  80
<212>  RNA
<213>  Homo sapiens

<400>  5
aacagcuaug gugaugauua gagacauacu aauguacaug augaccaugg augaaaaaua      60

uguaaacagc auuuggacc                                                    80

<210>  6
<211>  80
<212>  RNA
<213>  Homo sapiens

<400>  6
auaguauuga aucccacuag auuuggaacu acggugccau cagcaaugca gcaagucuug      60

gaaaaccuua cggagcugcc                                                  80

<210>  7
<211>  80
<212>  RNA
<213>  Homo sapiens

<400>  7
aggaggcaag ggagagagug gccgauugca ucacuaucag auaucagaaa accaggacuc      60

ugcagauguu gauccucagg                                                  80

<210>  8
<211>  80
<212>  RNA
<213>  Homo sapiens

<400>  8
cauugcuauu uuugcacuuc aguucacaua cuauuuaugg guuuaacuau agaucaacac      60

aucaucuugc aucucauggg                                                  80

<210>  9
<211>  80
<212>  RNA
<213>  Homo sapiens

<400>  9
ucucaccaaa gcccugggga augaaugacu uguucaggag ugacagauug caguacguau      60

uuuaggaccu gccacccauu                                                  80

<210>  10
<211>  80
<212>  RNA
<213>  Homo sapiens

<400>  10
gaaacaagug accaacaguu gaaucaaagu auggacacag guucgauaca agaggcuguu      60

uuccuagcgu ggcuugcugc                                                  80

<210>  11
<211>  80
<212>  RNA
<213>  Homo sapiens

<400>  11
ucugcaaagg aggagugcau gcuaauucag agaaauacag uucuuggaga agcugaauau      60

cuucucuuga gagagccagg                                                  80


<210>  12
<211>  80
<212>  RNA
<213>  Homo sapiens

<400>  12
ugaacaccug aagcucccca ucaagggaau gaacuucaca cuaaagccaa uucaaauacu      60

uucaaaucca cugucuacau                                                  80


<210>  13
<211>  80
<212>  RNA
<213>  Homo sapiens

<400>  13
uuuucgauaa ucuuuuggug uacugcaaaa gaaaacacag aaagacaagu uucguaucua      60

ugaugaauau uguaguaacc                                                  80


<210>  14
<211>  80
<212>  RNA
<213>  Homo sapiens

<400>  14
acuggacaug ucaguguccu uucacaccca ugugaaagag gucagccaag augggaaguc      60

gcuccuugac aagcuccagc                                                  80


<210>  15
<211>  80
<212>  RNA
<213>  Homo sapiens

<400>  15
uuuguaguau uuuagauugg uaucacagug caccugaaaa gcugaacuca cgggaaucaa      60

auggcguagg uacaauuuug                                                  80


<210>  16
<211>  80
<212>  RNA
<213>  Homo sapiens

<400>  16
aaaaccucca ccaccggcag cauccugaau cuuaaccugg augaaugaaa ucgaaguaug      60

uccagaaugu uaucuagcug                                                  80


<210>  17

<211>  200
<212>  DNA
<213>  Homo sapiens

<400>  17
ctttaccatt tatagttgga gttgactcaa ggtattttga tcttcatgac ccaccacaag      60

atgttgtttg cattgacttg gatacgaaca tgttatatgt agttctatat gaagggaaag      120

aacggcttat tccaggatgt gaagtgatcc tagccacacc ctatggtcgc tgtgccaatg      180

tcaacaatag ttcaactact                                                  200


<210>  18
<211>  200
<212>  DNA
<213>  Homo sapiens

<400>  18
acttgatcac tgagaagaga gttctaaaaa gctgaataaa aatttcattg caatcttcca      60

attcaaagca gatgttatat gatttaaccc aagctaaatt cgtccaagaa aacattgcca      120

aagaggacga ttctgtgttg ccaaatcaga atctttggag ccaaacaatt tagctagaag      180

tcgaacaaca gctaatcgct                                                  200


<210>  19
<211>  200
<212>  DNA
<213>  Homo sapiens

<400>  19
gccggaggac tatcgacagc agacctttga aaaaacagac ttgaaagtgc ctgccaaagc      60

caagctcacc aaactgaaga tgccttctca gtcagttgag tttgtacaat gacgacagaa      120

acctgcttcg aattagagag aaggaaagac gcaaccagga agcccaccaa gagaaagagg      180

catttcctga aaagattccc                                                  200


<210>  20
<211>  200
<212>  DNA
<213>  Homo sapiens

<400>  20
gaattgcatt ttttagctac tctagctcaa gagactgggg tgtggaaaaa cccggtactg      60

tgcactattc tttcccagga accattggat aaggataaag gcttgcccca gaacacacac      120

tatagagcag atgcagtagc cacaaataac tttgtgggga tctagtagct cccttaaaga      180

atgaggtgag gcaggctgaa                                                  200


<210>  21
<211>  200
<212>  DNA
<213>  Homo sapiens

<400>  21
cctcaaaact aagaccactg ttattcttac gctggatttc ttgaattgca tttttcagaa      60

ggtcccaaat gctgtttaca tatttttcat ccatggtcat catgtacatt agtatgtctc     120

taatcatcac catagctgtt tgatgatcat tccaagcttg atttagcgtt tgaagaaagt     180

tgttattcaa tgaatttagt                                                 200


<210>  22
<211>  200
<212>  DNA
<213>  Homo sapiens

<400>  22
aaaacattag tattgatagg agctcaaggt gtaggccgaa gaagcttgaa aaacaggttc      60

atagtattga atcccactag atttggaact acggtgccat cagcaatgca gcaagtcttg     120

gaaaacctta cggagctgcc ctcgtctact ggagcagaag aaatagacct aattttcctc     180

aagggaatta tggagaatcc                                                 200


<210>  23
<211>  200
<212>  DNA
<213>  Homo sapiens

<400>  23
ctctctcagc ctgttcttcc atttcagctt cttcatcaat agttcgagta aatgaatgct      60

cctgaggatc aacatctgca gagtcctggt tttctgatat ctgatagtga tgcaatcggc     120

cactctctcc cttgcctcct gtgtgtccaa cagtgcctaa accaaaacac cctgctagga     180

actgcagcct cacagacgtg                                                 200


<210>  24
<211>  200
<212>  DNA
<213>  Homo sapiens

<400>  24
tattgctatt gtaccaggct acatatctcg gtcagtagct ggatcctttg ataatgaagg      60

cattgctatt tttgcacttc agttcacata ctatttatgg gtttaactat agatcaacac     120

atcatcttgc atctcatggg ttctatgaat ttttaaattg gtttgatgaa agagcatggt     180

atccactagg aagaatagta                                                 200


<210>  25
<211>  200
<212>  DNA
<213>  Homo sapiens

<400>  25
gtgcagggct ttgatcatag agtggatttt caaacattgg agcaggaaga ggctcccgac      60

tctcaccaaa gccctgggga atgaatgact tgttcaggag tgacagattg cagtacgtat 120

tttaggacct gccacccatt ttgatttgaa agattatgtt gtcggaattt cagacactgc 180

aggtaaagat ctaattttac 200

<210> 26
<211> 200
<212> DNA
<213> Homo sapiens

<400> 26
agcagtctct tcacaactgg cggcgtgaat ggcaagatgg acgacatgtt cttaccaaag 60

gcagcaagcc acgctaggaa aacagcctct tgtatcgaac ctgtgtccat actttgattc 120

aactgttggt cacttgtttc tccatcttca ctgatatatc caggaggtgg cgtttctaca 180

aaagtttaaa acaaatcaag 200

<210> 27
<211> 200
<212> DNA
<213> Homo sapiens

<400> 27
ttagcaccat caaaatactg gaaatccaca tacactgtgt cgttgcttag tgctacatgg 60

cctggctctc tcaagagaag atattcagct tctccaagaa ctgtatttct ctgaattagc 120

atgcactcct cctttgcaga aaaatggctt ctgcttgaaa tgccaaagtc aaagcagtac 180

ttattcttcc ccatgtcaaa 200

<210> 28
<211> 200
<212> DNA
<213> Homo sapiens

<400> 28
gattattgtg gctcaccttg gctacctgaa ctacactcag tatacagtga tagtgggatt 60

tgaacacctg aagctcccca tcaagggaat gaacttcaca ctaaagccaa ttcaaatact 120

ttcaaatcca ctgtctacat acaatcagca actatggctg acatgtgttg ttgagttgga 180

tcaatcaaaa ggtatggagc 200

<210> 29
<211> 200
<212> DNA
<213> Homo sapiens

<400> 29
taatgtgtgg agtcttactg aaaatttctt ctggaaatat tcaagaacgg gtgtttttc 60

ttttcgataa tcttttggtg tactgcaaaa gaaaacacag aaagacaagt ttcgtatcta 120

tgatgaatat tgtagtaacc atgagaaggc acaaaaatta cttcttgaac tcaacaaaat 180

aagaacaatc cggacatttc 200

<210> 30
<211> 200
<212> DNA
<213> Homo sapiens

<400> 30
ccatcagctg gaagaccgga ttcaagattt cgttcggcgt gttgagcagc gaaagatcct 60

actggacatg tcagtgtcct ttcacaccca tgtgaaagag gtcagccaag atgggaagtc 120

gctccttgac aagctccagc ggcccttgac tcccggcagc tccgattccc tgacagcctc 180

tgccaactac tccaaggccg 200

<210> 31
<211> 200
<212> DNA
<213> Homo sapiens

<400> 31
acagaattgg atcatcttgg gctggggctg aaattatggg tccacagtcc ccatgccctc 60

caaaattgta cctacgccat ttgattcccg tgagttcagc ttttcaggtg cactgtgata 120

ccaatctaaa atactacaaa tctacaatgc tgctgcaaaa tggtatcatt catgttttca 180

aaatcaccag aattccatac 200

<210> 32
<211> 200
<212> DNA
<213> Homo sapiens

<400> 32
   aagcaaagta aatccaactt acacaaggct cacaaaacca gttatctcgt ttttggcaag 60
   cagctagata acattctgga catacttcga tttcattcat ccaggttaag attcaggatg 120
   ctgccggtgg tggaggtttt gtccgttttc gtcgtgatag gagctgacag ttgaggagag 180
   aagggctttg ggctgccgga 200

## Description

### FIELD OF THE INVENTION

The present invention associates multiple circular (circRNA) with key functions of endothelial cells. The present invention provides circRNA transcripts that are correlated with cardiovascular diseases, in particular acute myocardial infarction. The circRNA of the invention therefore serve as biomarkers in the diagnosis of cardiovascular disorders. The invention provides the new nucleic acid molecules as well as diagnostic kits and compositions comprising the nucleic acids. Modulation of the expression of the circRNA of the invention further leads to endothelial cell sprouting, and therefore is applied as a treatment for cardiovascular diseases or pathological angiogenesis. The invention provides therapeutic agents modulating circRNA expression or function.

### DESCRIPTION

RNA sequencing revealed that the human genome comprises about 20.000 protein coding genes, which is much less compared to more primitive organisms such as plants, despite a higher complexity of mammalian biology. Roughly 70% of the human genome, however, is transcribed and several classes of non-coding RNAs have been identified, which control tissue homeostasis and pathophysiological processes in the cardiovascular system. Alternative splicing represents an additional way to increase the complexity by modulating the sequential arrangement of exons and thus the biological properties of a given coding or non-coding gene. Thereby, particular exons of a gene may be included or excluded from the final processed messenger RNA. This leads to the generation of many protein isoforms with different biological properties as it, for example, was shown for VEGF-A, for which many spliced forms with different biological activities are known.

In addition to joining different exons to form linear transcripts, RNA molecules can be joined together by splicing reactions leading to the formation of circular RNAs (CircRNAs). CircRNAs can be generated from back-spliced exons or intron-derived RNA. Back-splicing has been considered as a rare event and was initially shown in plants. In mammalian cells, circRNAs were also reported already in the 90s, such as circular RNA transcripts of the dystrophin gene in the muscle. Recently, computational analysis of deep sequencing data provides evidences that the number of circRNAs is higher than thought, and thousands of circRNAs were identified. For example, 1,950 circRNAs were detected in HEK293 cells and human leukocytes, >25,000 circular transcripts are reported in fibroblasts, and 7,122 human circRNAs were annotated using data sets from the ENCODE project. A prominent example is the one derived from an antisense transcript of the sex-determining region Y (SRY) locus that is highly expressed in testis. Circular RNAs are also formed from the transcript ANRIL, a non-coding transcript of the 9q21 locus that is associated with the risk for atherosclerosis. However, little is known regarding regulation and function of circRNAs. Recent studies suggest that circRNAs may act as sponges for microRNAs. For example, a circular transcript deriving from CDR1as harbors 63 miR-7 binding sites and acts as sponge for this miR, and the circular RNA sponge for miR-7 (ciRS-7) harbors more than 70 conserved binding sites for miR-7. To obtain first insights into a putative role of circRNAs in vascular biology, we characterized the expressions and hypoxia-induced regulation of circRNAs in endothelial cells and showed that the circRNA cZNF292 is regulated by hypoxia in endothelial cells in vitro and increased in plasma of patients with acute myocardial infarction. Silencing of cZNF292 reduces angiogenic sprouting in vitro.

A critical role for non-coding RNA in endothelial cells is known. Repression of MALAT1 inhibited proliferation of endothelial cells and reduced neonatal retina vascularization and pharmacological inhibition of MALAT1 by GapmeRs reduced blood flow recovery and capillary density after hindlimb ischemia (Michalik et al. Circ Res. 2014 Apr 25;114(9):1389-97).

ANRIL is a long non-coding RNA which is transcribed from the INK/ARF locus and was shown to be expressed in tissues and cell types that are affected by atherosclerosis such as primary coronary smooth muscle cells, vascular endothelial cells, and human monocyte-derived macrophage cells. ANRIL transcripts were directly correlated with the severity of atherosclerosis (Holdt, et al. Arterioscler. Thromb. Vasc. Biol. 2010, 30, 620-627).

The lnc-Ang362 has been shown to be co expressed with to micro RNA (miR) that are correlated with cardiovascular events. The miR play a critical role in smooth muscle cell proliferation and neointimal hyperplasia in response to vascular injury. Interestingly, knockdown of Lnc-Ang362 reduces the expression of these miRNAs as well as cell growth (Davis, B.N et al. J. Biol. Chem. 2009, 284, 3728-3738).

In view of the state of the art it was therefore an object of the present invention to provide novel options for the treatment of diseases that are associated with endothelial dysfunction such as angiogenesis or cardiovascular disorders. The present invention seeks to provide new drug targets, therapeutics and diagnostics for these diseases based on comprehensive deep sequencing approach of the endothelial transcriptome in response to hypoxia.

### Nucleic Acids

The above problem is solved in a first aspect by a nucleic acid, comprising a sequence
a. Having at least 70%, 75%, 80%, 85%, 90%, 95%, 96%, 97%, 98%, 99% or most preferably 100% sequence identity to a circular RNA comprising a sequence shown in any of SEQ ID NO: 1 to 16,
b. Which is complementary to or binds to a sequence having at least 70%, 75%, 80%, 85%, 90%, 95%, 96%, 97%, 98%, 99% or most preferably 100% sequence identity to a circular RNA comprising a sequence shown in any of SEQ ID NO: 1 to 16,
c. Of at least 5 to 50 successive nucleic acid residues of a sequence having at least 70%, 75%, 80%, 85%, 90%, 95%, 96%, 97%, 98%, 99% or most preferably 100% sequence identity to a circular RNA comprising a sequence shown in any of SEQ ID NO: 1 to 16,
d. Which is complementary to or binds to a sequence of at least 5 to 50 successive nucleic acid residues of a sequence having at least 70%, 75%, 80%, 85%, 90%, 95%, 96%, 97%, 98%, 99% or most preferably 100% sequence identity to a circular RNA comprising a sequence shown in any of SEQ ID NO: 1 to 16.

Preferably the nucleic acids of the invention are provided as isolated nucleic acids.

In course of the present invention endothelial circRNAs were identified by computational analysis of next-generation sequencing data from ribominus RNA (preparation where ribosomal RNA was completely depleted) generated from human umbilical venous endothelial cells cultured under normoxic or hypoxic conditions. The herein firstly described circRNA are listed in table 1.

**Table 1: circRNA of the invention**

| **Name** | **Host Gene** | **Exons (Backsplicesite)** | **SEQ ID NO:** | **Chromosome Position (Back-splicesite GRCh37/hg19)** |
|---|---|---|---|---|
| U1 | DENND4C | Exon10/Exon3 | **1** | |
| U2 | PDS5A | Exon9/Exon5 | **2** | Chr4:39915230-39927553 |
| U3 | AFF1 | Exon5/Exon4 | **3** | Chr4:87967317-87968746 |
| U4 | ZNF292 | Exon4/Intron1 | **4** | Chr6:87920168-87928449 |
| U5 | CUL3 | Exon3/Exon2 | **5** | Chr2:225400244-225422573 |
| U6 | MPP6 | Exon10/Exon3 | **6** | Chr7:24663281-24708279 |
| U7 | HERC1 | Exon21/Exon26 | **7** | Chr15:63988322-64008672 |
| D1 | STT3B | Exon3/Exon2 | **8** | Chr3:31617887-31621588 |
| D2 | PTPR6 | Intron1/Intron1 | **9** | Chr3:61608136-61609229 |
| D3 | SMAD2 | Exon6/Exon2 | **10** | Chr18:45391429-45423180 |
| D4 | THSD1 | Exon3/Exon3 | **11** | Chr13:52971366-52972329 |
| D5 | TMEM181 | Exon6/Exon4 | **12** | Chr6:159004985-159010814 |
| D6 | PREX2 | Exon7/Exon4 | **13** | Chr8:68934270-68950527 |
| D7 | TRIO | Exon11/Exon9 | **14** | Chr5:14316621-14336836 |
| D8 | MED13L | Intron2/Exon2 | **15** | Chr12:116668237-116675510 |
| D9 | ZMYND8 | Exon15/Exon12 | **16** | Chr20:45891031-45912392 |

For the context of the present invention the circRNA molecules represented by comprising the sequences shown in SEQ ID NO 1 to 16 shall be recognized as circular molecules. In other words for each circRNA, the exons/introns of the respective circRNA are joined together in one circular RNA molecule. Therefore, terms such as "a nucleic acid comprising a sequence of a circRNA comprising a sequence of any of SEQ ID NO 1 to 16" shall be understood to refer to a nucleic acid - either linear or circular - that comprises a sequence of successive nucleic acid residues that can be found in any of the circular RNAs of the invention which are defined by comprising the sequences of SEQ ID NO 1 to 16. In alternative embodiments, instead of the shorter exemplary sequences of the circRNAs in SEQ ID NO: 1 to 16, one may also refer to the longer transcript sequences as provided in table 3 below, which refer to the same circRNAs and are depicted in SEQ ID NO: 17 to 32. These sequences are provided as cDNA and therefore contain thymine instead of uracil. In all aspects or embodiments of the invention, whenever referring to a circRNA comprising a sequence shown in any of SEQ ID NO: 1 to 16, alternatively the embodiments may refer to the SEQ ID NO 17 to 32.

"Nucleic acid" or a "nucleic acid molecule" as used herein refers to any DNA or RNA molecule, either single or double stranded and, if single stranded, the molecule of its complementary sequence in either linear or circular form. In discussing nucleic acid molecules, a sequence or structure of a particular nucleic acid molecule may be described herein according to the normal convention of providing the sequence in the 5' to 3' direction. With reference to nucleic acids of the invention, the term "isolated nucleic acid" is sometimes used. This term, when applied to DNA, refers to a DNA molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or host organism.

Preferably the isolated nucleic acid is a single stranded circular RNA, a nucleic acid probe, a nucleic acid primer or an antisense construct such as a siRNA or GapmeR.

The term "probe" as used herein refers to an oligonucleotide, polynucleotide or nucleic acid, either RNA or DNA, whether occurring naturally as in a purified restriction enzyme digest or produced synthetically, which is capable of annealing with or specifically hybridizing to a nucleic acid with sequences complementary to the probe. A probe may be either single stranded or double stranded. The exact length of the probe will depend upon many factors, including temperature, source of probe and use of the method. For example, for diagnostic applications, depending on the complexity of the target sequence, the oligonucleotide probe typically contains 15 25 or more nucleotides, although it may contain fewer nucleotides. The probes herein are selected to be "substantially" complementary to different strands of a particular target nucleic acid sequence. This means that the probes must be sufficiently complementary so as to be able to "specifically hybridize" or anneal with their respective target strands under a set of pre-determined conditions. Therefore, the probe sequence need not reflect the exact complementary sequence of the target. For example, a non-complementary nucleotide fragment may be attached to the 5' or 3' end of the probe, with the remainder of the probe sequence being complementary to the target strand. Alternatively, non-complementary bases or longer sequences can be interspersed into the probe, provided that the probe sequence has sufficient complementarity with the sequence of the target nucleic acid to anneal therewith specifically.

The term "primer" as used herein refers to an oligonucleotide, either RNA or DNA, either single stranded or double stranded, either derived from a biological system, generated by restriction enzyme digestion, or produced synthetically which, when placed in the proper environment, is able to functionally act as an initiator of template-dependent nucleic acid synthesis. When presented with an appropriate nucleic acid template, suitable nucleoside triphosphate precursors of nucleic acids, a polymerase enzyme, suitable cofactors and conditions such as a suitable temperature and pH, the primer may be extended at its 3' terminus by the addition of nucleotides by the action of a polymerase or similar activity to yield an primer extension product. The primer may vary in length depending on the particular conditions and requirement of the application. For example, in diagnostic applications, the oligonucleotide primer is typically 15 25 or more nucleotides in length. The primer must be of sufficient complementarity to the desired template to prime the synthesis of the desired extension product, that is, to be able anneal with the desired template strand in a manner sufficient to provide the 3' hydroxyl moiety of the primer in appropriate juxtaposition for use in the initiation of synthesis by a polymerase or similar enzyme. It is not required that the primer sequence represent an exact complement of the desired template. For example, a non complementary nucleotide sequence may be attached to the 5' end of an otherwise complementary primer. Alternatively, non complementary bases may be interspersed within the oligonucleotide primer sequence, provided that the primer sequence has sufficient complementarity with the sequence of the desired template strand to functionally provide a template primer complex for the synthesis of the extension product. The nucleic acid product obtainable by a PCR using two primers is referred to as an "amplicon".

In context of the herein described invention it is preferred that the nucleic acids of the invention, in particular, antisense constructs, primers, probe or amplicons of the invention of any given circRNA of the invention comprise, or are complementary to, the sequence of the back-splicing site of that circRNA according to table 2 below. Preferably the nucleic acid comprises or is complementary to the backsplicing site, more preferably including an additional 1, 2, 3, 4, 5, 6, 7, 8, 9, or 10 nucleic acids in upstream and downstream position of the back splicing site of a circRNA of the invention. A most preferred characteristic is therefore that any nucleic acid of the invention comprises or is complementary to a stretch of at least 10 nucleic acid residues, preferably 15, more preferably 20, successive nucleic acids comprising the backsplicing site of a circRNA.

In the context of the present invention the term "binds to" in the context of nucleic acids shall be understood as being able to hybridize with a another nucleic acid molecule based on the Watson-Crick base pairing, preferably to hybridize with a nucleic acid molecule under stringent conditions. The term "hybridize under stringent conditions", and grammatical equivalents thereof, refers to the ability of a nucleic acid molecule to hybridize to a target nucleic acid molecule (such as a target nucleic acid molecule immobilized on a DNA or RNA blot, such as a Southern blot or Northern blot) under defined conditions of temperature and salt concentration. Typically, stringent hybridization conditions are no more than 25°C to 30°C (for example, 10°C) below the melting temperature (Tₘ) of the native duplex. By way of nonlimiting example, representative salt and temperature conditions for achieving stringent hybridization are: 5×SSC, at 65°C, or equivalent conditions; see generally, Sambrook et al. Molecular Cloning. A Laboratory Manual, 2nd ed., Cold Spring Harbor Press, 1987; Ausubel et al., Current Protocols in Molecular Biology, Greene Publishing, 1987. Tₘ for nucleic acid molecules greater than about 100 bases can be calculated by the formula Tₘ=0.5+0.41%(G+C)-log (Na+). For oligonucleotide molecules less than 100 bases in length, exemplary hybridization conditions are 5 to 10° C. below Tₘ. On average, the Tm of a short oligonucleotide duplex is reduced by approximately (500/oligonucleotide length)°C.

The term "sequence identity" or "percent identical" as applied to nucleic acid molecules is the percentage of nucleic acid residues in a candidate nucleic acid molecule sequence that are identical with a subject nucleic acid molecule sequence, after aligning the sequences to achieve the maximum percent identity, and not considering any nucleic acid residue substitutions as part of the sequence identity. No gaps are introduced into the candidate nucleic acid sequence in order to achieve the best alignment.

When assessing the identity or complementarity of a first and second nucleic acid sequence wherein one sequence is a DNA sequence and the other is an RNA sequence, it must be borne in mind that RNA sequences comprise uracil whereas DNA sequences would comprise thymine as complementary base of adenine. Therefore, whenever in context of the present invention a RNA sequence is referred to any of the sequences in the sequence protocol of SEQ ID NO 17 to 32 or in table 3 below, these sequences have to be converted into RNA by exchanging thymine with uracil. When assessing sequence identity, an uracil residue is considered to be identical to a thymine residue (because both bind adenine) and when assessing complementarity a uracil residue is considered to be complementary to/capable of forming hydrogen bonds with an adenine residue. In this respect, the present invention when referring to a nucleic acid which is RNA in accordance with the invention contains uracil bases instead of thymine bases, and *vice versa.* This difference is well recognized in the pertinent art and easily recognized by a person of skill.

As used herein, the term "RNA interference" ("RNAi") or "antisense technology" refers to a selective targeted intracellular degradation of RNA. RNAi occurs in cells naturally to remove foreign RNAs (e.g., viral RNAs). Natural RNAi proceeds via fragments cleaved from free dsRNA which direct the degradative mechanism to other similar RNA sequences. Alternatively, RNAi can be initiated by the hand of man, for example, to silence the expression of endogenous target genes, such as PKC-zeta.

As used herein, the term "small interfering RNA" ("siRNA") (also referred to in the art as "short interfering RNAs") refers to an RNA (or RNA analog) comprising between about 10-50 nucleotides (or nucleotide analogs) which is capable of directing or mediating targeted RNA interference.

Aspects of the present invention relate to various vehicles comprising the nucleic acid molecules, preferably the antisense or circRNA molecules, of the present invention. By vehicle is understood an agent with which genetic material can be transferred. Herein such vehicles are exemplified as nucleic acid constructs, vectors, and delivery vehicles such as viruses and cells.

By nucleic acid construct or expression construct is understood a genetically engineered nucleic acid. The nucleic acid construct may be a non-replicating and linear nucleic acid, a circular expression vector, an autonomously replicating plasmid or viral expression vector. A nucleic acid construct may comprise several elements such as, but not limited to genes or fragments of same, promoters, enhancers, terminators, poly-A tails, linkers, markers and host homologous sequences for integration. Methods for engineering nucleic acid constructs are well known in the art (see, e.g., Molecular Cloning: A Laboratory Manual, Sambrook et al., eds., Cold Spring Harbor Laboratory, 2nd Edition, Cold Spring Harbor, N.Y., 1989).

Several nucleic acid molecules may be encoded within the same construct and may be linked by an operative linker. By the term operative linker is understood a sequence of nucleotides two parts of a nucleic acid construct in a manner securing the biological processing of the encoded nucleic acid molecules.

The term promoter will be used here to refer to a group of transcriptional control modules that are clustered around the initiation site for RNA polymerase. The best known example of this is the TATA box, but in some promoters lacking a TATA box, such as the promoter for the mammalian terminal deoxynucleotidyl transferase gene and the promoter for the SV 40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. Depending on the promoter, it appears that individual elements can function either cooperatively or independently to activate transcription. Any promoter that can direct transcription initiation of the sequences encoded by the nucleic acid construct may be used in the invention.

An aspect of the present invention comprises the nucleic acid construct wherein the sequence of at least one nucleic acid molecule is preceded by a promoter enabling expression of the at least one nucleic acid molecule.

It is a further aspect that the promoter is selected from the group of constitutive promoters, inducible promoters, organism specific promoters, tissue specific promoters and cell type specific promoters. Examples of promoters include, but are not limited to: constitutive promoters such as: simian virus 40 (SV40) early promoter, a mouse mammary tumour virus promoter, a human immunodeficiency virus long terminal repeat promoter, a Moloney virus promoter, an avian leukaemia virus promoter, an Epstein-Barr virus immediate early promoter, a Rous sarcoma virus (RSV) promoter, a human actin promoter, a human myosin promoter, a human haemoglobin promoter, cytomegalovirus (CMV) promoter and a human muscle creatine promoter, inducible promoters such as: a metallothionine promoter, a glucocorticoid promoter, a progesterone promoter, and a tetracycline promoter (tet-on or tet-off), tissue specific promoters such as: HER-2 promoter and PSA associated promoter.

An aspect of the present invention comprises the nucleic acid construct as described in any of the above, comprised within a delivery vehicle referred to as vector. A delivery vehicle is an entity whereby a nucleotide sequence can be transported from at least one media to another. Delivery vehicles are generally used for expression of the sequences encoded within the nucleic acid construct and/or for the intracellular delivery of the construct. It is within the scope of the present invention that the delivery vehicle is a vehicle selected from the group of: RNA based vehicles, DNA based vehicles/vectors, lipid based vehicles, virally based vehicles and cell based vehicles. Examples of such delivery vehicles include, but are not limited to: biodegradable polymer microspheres, lipid based formulations such as liposome carriers, coating the construct onto colloidal gold particles, lipopolysaccharides, polypeptides, polysaccharides, pegylation of viral vehicles.

A preferred embodiment of the present invention comprises a virus as a delivery vehicle, where the virus is selected from the non-exhaustive group of: adenoviruses, retroviruses, lentiviruses, adeno-associated viruses, herpesviruses, vaccinia viruses, foamy viruses, cytomeg-aloviruses, Semliki forest virus, poxviruses, RNA virus vector and DNA virus vector. Such viral vectors are well known in the art.

For the inhibition of the circRNA of the invention, it is in certain embodiments preferred to use antisense oligonucleotides in order to impair the circRNA expression or function. Antisense oligonucleotides (ASOs) are synthetic nucleic acids that bind to a complementary target and suppress function of that target. Typically ASOs are used to reduce or alter expression of RNA targets - circRNA is one preferred example of an RNA that can be targeted by ASOs. As a general principle, ASOs can suppress RNA function via two different mechanisms of action: 1) by steric blocking, wherein the ASO tightly binds the target nucleic acid and inactivates that species, preventing its participation in cellular activities, or 2) by triggering degradation, wherein the ASO binds the target and leads to activation of a cellular nuclease that degrades the targeted nucleic acid species. One class of "target degrading". ASOs may be composed of several types of nucleic acids, such as RNA, DNA or PNA.

In order to enhance the half-life of an ASO, modifications of the nucleic acids can be introduced. It is in particular useful to protect the ASO from degradation by cellular endonucleases. One modification that gained widespread use comprised DNA modified with phosphorothioate groups (PS). PS modification of internucleotide linkages confers nuclease resistance, making the ASOs more stable both in serum and in cells. As an added benefit, the PS modification also increases binding of the ASO to serum proteins, such as albumin, which decreases the rate of renal excretion following intravenous injection, thereby improving pharmacokinetics and improving functional performance. Therefore, PS modified ASOs are encompassed by the present invention.

Further modifications target the 3 '-end of an ASO molecule, for example "Gapmer" compounds having 2'-methoxyethylriboses (MOE's) providing 2'-modified "wings" at the 3' and 5' ends flanking a central 2'-deoxy gap region. ASO modifications that improve both binding affinity and nuclease resistance typically are modified nucleosides including locked nucleic acids (LNA), wherein a methyl bridge connects the 2'- oxygen and the 4'-carbon, locking the ribose in an A-form conformation; variations of LNA are also available, such as ethylene-bridged nucleic acids (ENA) that contain an additional methyl group, amino-LNA and thio-LNA. Additionally, other 2'-modifications, such as 2'-0- methoxyethyl (MOE) or 2'-fluoro (2'-F), can also be incorporated into ASOs. Such exemplary modifications are comprised by the present invention.

In the context of the present invention this means that the term "antisense oligonucleotide" pertains to a nucleotide sequence that is complementary to at least a portion of a target circRNA sequence of the invention. The term "oligonucleotide" refers to an oligomer or polymer of nucleotide or nucleoside monomers consisting of naturally occurring bases, sugars, and intersugar (backbone) linkages. The term also includes modified or substituted oligomers comprising non-naturally occurring monomers or portions thereof, which function similarly. Such modified or substituted oligonucleotides may be preferred over naturally occurring forms because of properties such as enhanced cellular uptake, or increased stability in the presence of nucleases as already mentioned above. The term also includes chimeric oligonucleotides which contain two or more chemically distinct regions. For example, chimeric oligonucleotides may contain at least one region of modified nucleotides that confer beneficial properties (e.g. increased nuclease resistance, increased uptake into cells) as well as the antisense binding region. In addition, two or more antisense oligonucleotides may be linked to form a chimeric oligonucleotide.

The antisense oligonucleotides of the present invention may be ribonucleic or deoxyribonucleic acids and may contain naturally occurring bases including adenine, guanine, cytosine, thymidine and uracil. The oligonucleotides may also contain modified bases such as xanthine, hypoxanthine, 2-aminoadenine, 6-methyl, 2-propyl and other alkyl adenines, 5-halo uracil, 5-halo cytosine, 6-aza thymine, pseudo uracil, 4-thiouracil, 8-halo adenine, 8-aminoadenine, 8-thiol adenine, 8-thiolalkyl adenines, 8-hydroxyl adenine and other 8-substituted adenines, 8-halo guanines, 8-amino guanine, 8-thiol guanine, 8-thiolalkyl guanines, 8-hydrodyl guanine and other 8-substituted guanines, other aza and deaza uracils, thymidines, cytosines, adenines, or guanines, 5-tri-fluoromethyl uracil and 5-trifluoro cytosine.

Other antisense oligonucleotides of the invention may contain modified phosphorous, oxygen heteroatoms in the phosphate backbone, short chain alkyl or cycloalkyl intersugar linkages or short chain heteroatomic or heterocyclic intersugar linkages. For example, the antisense oligonucleotides may contain phosphorothioates, phosphotriesters, methyl phosphonates and phosphorodithioates. In addition, the antisense oligonucleotides may contain a combination of linkages, for example, phosphorothioate bonds may link only the four to six 3'-terminal bases, may link all the nucleotides or may link only 1 pair of bases.

The antisense oligonucleotides of the invention may also comprise nucleotide analogs that may be better suited as therapeutic or experimental reagents. An example of an oligonucleotide analogue is a peptide nucleic acid (PNA) in which the deoxribose (or ribose) phosphate backbone in the DNA (or RNA), is replaced with a polymide backbone which is similar to that found in peptides. PNA analogues have been shown to be resistant to degradation by enzymes and to have extended lives in vivo and in vitro. PNAs also form stronger bonds with a complementary DNA sequence due to the lack of charge repulsion between the PNA strand and the DNA strand. Other oligonucleotide analogues may contain nucleotides having polymer backbones, cyclic backbones, or acyclic backbones. For example, the nucleotides may have morpholino backbone structures. Oligonucleotide analogues may also contain groups such as reporter groups, protective groups and groups for improving the pharmacokinetic properties of the oligonucleotide. Antisense oligonucleotides may also incorporate sugar mimetics as will be appreciated by one of skill in the art.

Antisense nucleic acid molecules may be constructed using chemical synthesis and enzymatic ligation reactions using procedures known in the art based on a given circRNA sequence such as those provided herein. The antisense nucleic acid molecules of the invention, or fragments thereof, may be chemically synthesized using naturally occurring nucleotides or variously modified nucleotides designed to increase the biological stability of the molecules or to increase the physical stability of the duplex formed with mRNA or the native gene, e.g. phosphorothioate derivatives and acridine substituted nucleotides. The antisense sequences may also be produced biologically. In this case, an antisense encoding nucleic acid is incorporated within an expression vector that is then introduced into cells in the form of a recombinant plasmid, phagemid or attenuated virus in which antisense sequences are produced under the control of a high efficiency regulatory region, the activity of which may be determined by the cell type into which the vector is introduced.

In another embodiment, siRNA technology may be applied to inhibit expression of a circRNA of the invention. Application of nucleic acid fragments such as siRNA fragments that correspond with regions in circRNA gene and which selectively target a circRNA may be used to block circRNA expression or function.

SiRNA, small interfering RNA molecules, corresponding to a region in the circRNA sequence are made using well-established methods of nucleic acid syntheses as outlined above with respect to antisense oligonucleotides. Since the structure of target circRNAs is known, fragments of RNA/DNA that correspond therewith can readily be made. The effectiveness of selected siRNA to impair circRNA function or expression, for example via targeted degradation, can be confirmed using a circRNA-expressing cell line. Briefly, selected siRNA may be incubated with a circRNA-expressing cell line under appropriate growth conditions. Following a sufficient reaction time, i.e. for the siRNA to bind with circRNA to result in decreased lev-els of the circRNA, the reaction mixture is tested to determine if such a decrease has occurred, for example via quantitative PCR, northern blotting etc.

Antisense oligonucleotides in accordance with the invention may comprise at least one modification that is incorporated at the terminal end of an antisense oligonucleotide, or between two bases of the antisense oligonucleotide, wherein the modification increases binding affinity and nuclease resistance of the antisense oligonucleotide. In one embodiment, the antisense oligonucleotide comprises at least one modification that is located within three bases of a terminal nucleotide. In another embodiment, the antisense oligonucleotide comprises at least one modification that is located between a terminal base and a penultimate base of either the 3'- or the 5 '-end of the oligonucleotide. In another embodiment, the antisense oligonucleotide comprises a modification at a terminal end of the oligonucleotide. In a further embodiment, the antisense oligonucleotide comprises a modification at the terminal end or between the terminal base and the penultimate base of both the 3'- and the 5'- ends of the antisense oligonucleotide. In yet a further embodiment, the oligonucleotide contains a non-base modifier at a terminal end or between the terminal base and the penultimate base at the 5 '-end and at the 3 '-end.

On the other hand, the circRNA of the invention may be directly used as an agonist of their own function. CircRNA can be expressed via genetic constructs as mentioned herein above, or alternatively, directly used. Therefore, circRNA of the invention are provided in their native form as well as chemically modified versions thereof. Chemically modified RNA (mo-dRNA) that can be used for the direct delivery of an circRNA sequence of the invention ( Zangi L. et al, "Modified mRNA directs the fate of heart progenitor cells and induces vascular regeneration after myocardial infarction" 2013, Nat Biotechnol). Such modified RNA may be produced by use of 3'-O-Me-m7G(5')ppp(5')G cap analogs and is described in Warren L, Manos PD, Ahfeldt T, et al. Highly efficient reprogramming to pluripotency and directed differentiation of human cells with synthetic modified mRNA. Cell Stem Cell. 2011;7:618-630.

### Diagnostics

In the context of the herein described invention it was surprisingly found that circRNAs were increased in plasma of patients with acute myocardial infarction and therefore are interesting new biomarkers of cardiovascular diseases. Therefore, the above problem of the invention is solved in a second aspect by a method of predicting, monitoring and/or diagnosing a cardiovascular disease in a subject, comprising the steps of:
a. Providing a biological sample from the subject,
b. Determining the level of one or more non-coding circular RNA transcripts (circRNA) in the biological sample, wherein the circRNA transcript is selected from a circRNA comprising a sequence shown in any of SEQ ID NO: 1 to 16;
c. Comparing the level of the circRNA in the biological sample to a control level of the one or more circRNA transcripts;
d. Predicting, monitoring and/or diagnosing the subject as having the cardiovascular disease when the level of the one or more circRNA in the biological sample is higher or lower compared to the control level.

As used herein, the term "level" or "expression level" refers to the expression level data that can be used to compare the expression levels of different circRNA among various samples and/or subjects as mentioned in the above method. In particular determining the level of a circRNA means the determination of the amount or concentration of a circRNA transcript. The term "amount" or "concentration" of certain circRNA refers respectively to the effective (i.e. total circRNA amount measured) or relative amount (i.e. total circRNA amount measured in relation to the sample size used) of the circRNA in a certain sample.

In some embodiments of the invention the method is an *in vitro.* or *ex vivo* method.

In preferred embodiments the diagnostic circRNA of the invention is selected from a circRNA comprising a sequence shown in SEQ ID NO: 1 to 4, and 11, preferably selected from a circRNA comprising a sequence shown in SEQ ID NO: 1, SEQ ID NO: 2, or SEQ ID NO: 4.

A cardiovascular disease in context of the present invention may be a disease associated with a pathological repressed endothelial cell repair, cell growth and/or cell division or is a disease treatable by improving endothelial cell repair, cell growth and/or cell division. Generally, the term "cardiovascular disease," as used herein, is intended to refer to all pathological states leading to a narrowing and/or occlusion of blood vessels throughout the body. In particular, the term "cardiovascular disease" refers to conditions including atherosclerosis, thrombosis and other related pathological states, especially within arteries of the heart and brain. Accordingly, the term "cardiovascular disease" encompasses, without limitation, various types of heart disease, as well as Alzheimer's disease and vascular dimension. However, preferably the cardiovascular disease is selected atherosclerosis, a coronary heart disease, an acute coronary symptom, preferably unstable angina pectoris or acute myocardial infarction (AMI), stable angina pectoris, stroke, preferably ischemic stroke, inflammation or autoimmune disease associated artheriosclerosis or restenosis.

In a broader sense a "cardiovascular disease" may also refer to a condition selected from the group consisting of acute coronary syndrome, acute lung injury (ALI), acute myocardial infarction (AMI), acute respiratory distress syndrome (ARDS), arterial occlusive disease, arteriosclerosis, articular cartilage defect, aseptic systemic inflammation, atherosclerotic cardiovascular disease, autoimmune disease, bone fracture, bone fracture, brain edema, brain hypoperfusion, Buerger's disease, burns, cancer, cardiovascular disease, cartilage damage, cerebral infarct, cerebral ischemia, cerebral stroke, cerebrovascular disease, chemotherapy-induced neuropathy, chronic infection, chronic mesenteric ischemia, claudication, congestive heart failure, connective tissue damage, contusion, coronary artery disease (CAD), critical limb ischemia (CLI), Crohn's disease, deep vein thrombosis, deep wound, delayed ulcer healing, delayed wound-healing, diabetes (type I and type II), diabetic neuropathy, diabetes induced ischemia, disseminated intravascular coagulation (DIC), embolic brain ischemia, frost-bite, graft-versus-host disease, hereditary hemorrhagic telengiectasiaischemic vascular disease, hyperoxic injury, hypoxia, inflammation, inflammatory bowel disease, inflammatory disease, injured tendons, intermittent claudication, intestinal ischemia, ischemia, ischemic brain disease, ischemic heart disease, ischemic peripheral vascular disease, ischemic placenta, ischemic renal disease, ischemic vascular disease, ischemic-reperfusion injury, laceration, left main coronary artery disease, limb ischemia, lower extremity ischemia, myocardial infarction, myocardial ischemia, organ ischemia, osteoarthritis, osteoporosis, osteosarcoma, Parkinson's disease, peripheral arterial disease (PAD), peripheral artery disease, peripheral ischemia, peripheral neuropathy, peripheral vascular disease, pre-cancer, pulmonary edema, pulmonary embolism, remodeling disorder, renal ischemia, retinal ischemia, retinopathy, sepsis, skin ulcers, solid organ transplantation, spinal cord injury, stroke, subchondral-bone cyst, thrombosis, thrombotic brain ischemia, tissue ischemia, transient ischemic attack (TIA), traumatic brain injury, ulcerative colitis, vascular disease of the kidney, vascular inflammatory conditions, von Hippel-Lindau syndrome, or wounds to tissues or organs.

In order to conduct the method of the invention step b. may involve the use of at least one isolated nucleic acid molecule according to above described embodiments relating to isolated nucleic acids. Preferably such isolated nucleic acids molecule is a primer and/or a probe. Furthermore it is preferred that the isolation of a nucleic acid is the isolation of RNA from the biological sample.

The methods of the invention may preferably include a further step of removing ribosomal and/or poly A RNA from the biological sample before determining the level of a circRNA of the invention. Thus, before determining the level of the circRNA of the invention, the biological sample or the isolated RNA therefrom is treated to remove any ribosomal and/or poly-A tail RNA (mRNA). Methods to remove rRNA or polyA RNA are well known in the art and are exemplary described in the example section of the present application. By removing any non circRNA from the sample, the results are more specific and sensitive due to a reduced RNA background "noise". This step may be performed in any aspect or embodiment of the invention which requires the detection or isolation of a circRNA of the invention.

Methods for the detection or nucleic acid molecules such as circRNA in a sample comprise PCR based methods which involve the target specific amplification of nucleic acids. However, any nucleic acid detection assay known to the skilled artisan may be used to detect one or more circRNA of the invention. As used herein, the term "nucleic acid detection assay" refers to any method of determining the nucleotide composition of a nucleic acid of interest. Nucleic acid detection assay include but are not limited to, sequencing methods, probe hybridization methods, structure specific cleavage assays (e.g., the INVADER assay, (Hologic, Inc); enzyme mismatch cleavage methods; polymerase chain reaction (PCR); branched hybridization methods; rolling circle replication; NASBA; molecular beacon technology; E-sensor technology (Motorola, U.S. Pat. Nos. 6,248,229); cycling probe technology; Dade Behring signal amplification methods; ligase chain reaction; and sandwich hybridization methods.

The term "subject" in context of the present invention in all of its embodiments and aspects is a mammal, preferably a human.

The term "biological sample" is a tissue sample, for example heart tissue sample, or a liquid sample, preferably a blood sample such as a whole blood sample, serum sample, or plasma sample. In context of the present invention the biological sample may be a venous or aortic plasma sample.

The term "control level" in context of the diagnostics of the invention corresponds to (i) the level of the one or more circRNA in a biological sample from a subject not suffering from, or not being at risk of developing, the cardiovascular disease, or (ii) the level of the one or more circRNA in a biological sample from the same subject at a different time point, for example before or after conducting a medical treatment.

Most preferred embodiments of the diagnostics described in context of the invention pertain to the above method wherein a higher level of a circRNA comprising the sequence shown in SEQ ID NO: 2 in a sample of venous plasma of the subject compared to the control level indicates acute myocardial infarction. Alternatively preferred is a method of the invention wherein a higher level of a circRNA comprising the sequence shown in SEQ ID NO: 1 and/or SEQ ID NO: 4 in a sample of aortic plasma of the subject compared to the control level indicates acute myocardial infarction.

### Therapeutics

A third aspect of the present invention then provides compounds useful as therapeutics in the treatment of diseases associated with a dysfunction of an endothelial cell. Provided is a compound for use in the treatment of a disease associated with endothelial cell dysfunction, wherein the compound is an agonist or is an antagonist of a non-coding circular RNA (circRNA) comprising a sequence shown in any of SEQ ID NO: 1 to 16.

The present invention is based on the development of circRNA as drug targets. Therefore, the third aspect of the invention pertains to the modulation of the expression or function of endothelial circRNA as disclosed herein above (table 1), preferably in the context of a medical treatment. For means or compounds enhancing the expression or function of a circRNA the present invention will refer to such means or compounds as "agonists" of a respective circRNA. Alternatively, the expression and/or function of a circRNA may be reduced or inhibited. In this case the present invention will refer to these mediators of the effect as "antagonists" of the respective circRNA of the invention. Since circRNA are RNA-molecules which mediate their biological activity either in the cellular cytoplasm or in the cell nucleus the person of skill can harness all known methods that intervene with the natural RNA metabolism.

Alternatively the present invention provides expression constructs of circRNA as agonists of the invention. A circRNA expression construct of the invention comprises preferably an expressible sequence of circRNA of the invention, optionally of homo logs thereof, operatively linked to a promoter sequence. Since expression constructs may be used both to express an agonists or an antagonist of a circRNA of the invention a detailed description of expression constructs us provided herein below. Reference is made to the above described isolated nucleic acids of the invention.

In order to impair circRNA expression/function in accordance with the herein described invention the person of skill may choose any suitable methodology for inhibiting RNA expression. In particular preferred are antisense approaches, which apply sequence complementary nucleic acid polymers (antagonists) which mediate the inhibition or destruction of a target RNAs.

According to some embodiments, an circRNA of the invention may be targeted using an inhibiting agent or therapeutic - an antagonist of the circRNA - targeting strategy such as antisense RNA, RNA interference (RNAi), siRNA, esiRNA, shRNA, miRNA, decoys, RNA aptamers, small molecule inhibitors, RNA/DNA-binding proteins/peptides, GapmeRs, LNA molecules or other compounds with different formulations to inhibit one or more physiological actions effected by circRNA. The antisense antagonists of the invention may either be directly administered or used, or alternatively, may be expressed using an expression construct, for example a construct expressing a miRNA having a sequence complementary to a circRNA of the invention.

The term "endothelial cell dysfunction" refers to a physiological dysfunction of normal biochemical processes carried out by endothelial cells, the cells that line the inner surface of all blood vessels including arteries and veins. For example, endothelial cell dysfunction may result in an inability of blood vessels, such as arteries and arterioles, to dilate normally in response to an appropriate stimulus.

In one preferred embodiment the disease associated with endothelial cell dysfunction is pathological angiogenesis, and wherein the compound is
a. An agonist of a circRNA, wherein the circRNA comprises a sequence shown in SEQ ID NO: 2 and/or 9, or
b. An antagonist of a circRNA, wherein the circRNA comprises a sequence shown in SEQ ID NO: 4 and/or 14.

The term "pathological angiogenesis" refers to the excessive formation and growth of blood vessels during the maintenance and the progression of several disease states. Examples where pathological angiogenesis can occur are blood vessels (atherosclerosis, hemangioma, hemangioendothelioma), bone and joints (rheumatoid arthritis, synovitis, bone and cartilage destruction, osteomyelitis, pannus growth, osteophyte formation, neoplasms and metastasis), skin (warts, pyogenic granulomas, hair growth, Kaposi's sarcoma, scar keloids, allergic oedema, neoplasms), liver, kidney, lung, ear and other epithelia (inflammatory and infectious processes (including hepatitis, glomerulonephritis, pneumonia), asthma, nasal polyps, otitis, transplantation, liver regeneration, neoplasms and metastasis), uterus, ovary and placenta (dysfunctional uterine bleeding (due to intrauterine contraceptive devices), follicular cyst formation, ovarian hyperstimulation syndrome, endometriosis, neoplasms), brain, nerves and eye (retinopathy of prematurity, diabetic retinopathy, choroidal and other intraocular disorders, leukomalacia, neoplasms and metastasis), heart and skeletal muscle due to work overload, adipose tissue (obesity), endocrine organs (thyroiditis, thyroid enlargement, pancreas transplantation), hematopoiesis (AIDS (Kaposi), hematologic malignancies (leukemias, etc.), tumour induced new blood vessels. The pathological angiogenesis may occur in connection with a proliferative disorder, most preferably in connection with a cancer disease. A cancer may be selected from the group consisting of liver cancer, lung cancer, breast cancer, colorectal cancer, stomach cancer and melanoma, most preferably wherein the cancer is solid cancer, even more preferably a metastatic solid cancer.

Alternatively, the invention provides a therapeutic compound for use in the treatment of a cardiovascular disease, and wherein the compound is
a. An antagonist of a circRNA, wherein the circRNA comprises a sequence shown in SEQ ID NO: 2 and/or 9, or
b. An agonist of a circRNA, wherein the circRNA comprises a sequence shown in SEQ ID NO: 4 and/or 14.

A cardiovascular disease is a disease as described herein above.

Preferably the circRNA agonist of the invention is selected from an isolated nucleic acid according to the above described first aspect of the invention, or an circRNA expression construct comprising a nucleic acid as described herein before, or a molecule that when contacted with a cell induces or increases the expression and or function of the circRNA.

A preferred antagonist is an antisense construct comprising a sequence that is complementary to a circRNA sequence of a circRNA comprising a sequence shown in any of SEQ ID NO 1 to 16, or is complimentary to a sequence having at least 70% sequence identity to a circRNA sequence of a circRNA comprising a sequence shown in any of SEQ ID NO: 1 to 16. Generally, the term "treatment" involves the administration of the compound to a subject suffering from, or suspected to be in danger of suffering from the disease associated with endothelial cell dysfunction.

The agonists or antagonists as described herein above are useful in the treatment of the various diseases mentioned above. Therefore the present invention provides the use of the compounds of the invention in a curative or prophylactic medical treatment involving the administration of a therapeutically effective amount of the compound to a subject in need of such a treatment.

The agonists or antagonists described may be used alone or in combination with other methods for treating of the various diseases associated with endothelial cell dysfunction. For example if a subject has been diagnosed with for example ischemia, the one or more agents described above may be combined with administration of a therapeutically effective amount of a compound that is therapeutically active for the treatment of an ischemic event.

The term "effective amount" as used herein refers to an amount of a compound that produces a desired effect. For example, a population of cells may be contacted with an effective amount of a compound to study its effect *in vitro.* (e.g., cell culture) or to produce a desired therapeutic effect *ex vivo* or *in vitro.* An effective amount of a compound may be used to produce a therapeutic effect in a subject, such as preventing or treating a target condition, alleviating symptoms associated with the condition, or producing a desired physiological effect. In such a case, the effective amount of a compound is a "therapeutically effective amount," "therapeutically effective concentration" or "therapeutically effective dose." The precise effective amount or therapeutically effective amount is an amount of the composition that will yield the most effective results in terms of efficacy of treatment in a given subject or population of cells. This amount will vary depending upon a variety of factors, including but not limited to the characteristics of the compound (including activity, pharmacokinetics, pharmacodynamics, and bioavailability), the physiological condition of the subject (including age, sex, disease type and stage, general physical condition, responsiveness to a given dosage, and type of medication) or cells, the nature of the pharmaceutically acceptable carrier or carriers in the formulation, and the route of administration. Further an effective or therapeutically effective amount may vary depending on whether the compound is administered alone or in combination with another compound, drug, therapy or other therapeutic method or modality. One skilled in the clinical and pharmacological arts will be able to determine an effective amount or therapeutically effective amount through routine experimentation, namely by monitoring a cell's or subject's response to administration of a compound and adjusting the dosage accordingly. For additional guidance, see Remington: The Science and Practice of Pharmacy, 21st Edition, Univ. of Sciences in Philadelphia (USIP), Lippincott Williams & Wilkins, Philadelphia, Pa., 2005, which is hereby incorporated by reference as if fully set forth herein.

The term "in combination" or "in combination with," as used herein, means in the course of treating the same disease in the same patient using two or more agents, drugs, treatment regimens, treatment modalities or a combination thereof, in any order. This includes simultaneous administration, as well as in a temporally spaced order of up to several days apart. Such combination treatment may also include more than a single administration of any one or more of the agents, drugs, treatment regimens or treatment modalities. Further, the administration of the two or more agents, drugs, treatment regimens, treatment modalities or a combination thereof may be by the same or different routes of administration.

The term "subject" as used herein means a human or other mammal. In some embodiments, the subject may be a patient suffering or in danger of suffering from a disease as disclosed herein.

### Diagnostic Kits

The present invention furthermore provides diagnostic kits comprising any of the herein disclosed compounds or compositions. The term "diagnostic kit" includes different packages of diagnostic and therapeutic compounds and reagents which are necessary for the implementation of the invention, as a rule, instructions for using the reagents. Preferred is a diagnostic kit that comprises one or more isolated nucleic acid molecules as described herein. Preferably the kit comprises one or more primers or probes suitable for the detection of any one of the herein described circRNA comprising a sequence shown in any of SEQ ID NO: 1 to 16. A kit of this invention also contains "instruction for use" of the materials contained in the package.

### Pharmaceutical compositions

In one embodiment, the composition comprises a compound, combination or composition as described herein, optionally together with a pharmaceutically acceptable carrier.

As used herein the language "pharmaceutically acceptable carrier" is intended to include any and all solvents, solubilizers, fillers, stabilizers, binders, absorbents, bases, buffering agents, lubricants, controlled release vehicles, nanoparticles, liposomes, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injection use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phos-phate buffered saline (PBS). In all cases, the injectable composition should be sterile and should be fluid to the extent that easy syringability exists. It must be stable under the condi-tions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the inject-able compositions can be brought about by including in the composition an agent which delays absorption, for example, aluminum monostearate and gelatin.

Sterile injectable solutions can be prepared by incorporating the active compound (e.g., a neuregulin) in the required amount in an appropriate solvent with one or a combination of ingredi-ents enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active in-gredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or or-ange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) or nanoparticles, including those prepared with poly(dl-lactide-co-glycolide), can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

Finally provided is the use of an agonist or antagonist of a circRNA of the invention, in the modulation of endothelial cell function.

The present invention will now be further described in the following examples with reference to the accompanying figures and sequences, nevertheless, without being limited thereto. For the purposes of the present invention, all references as cited herein are incorporated by reference in their entireties. In the Figures:

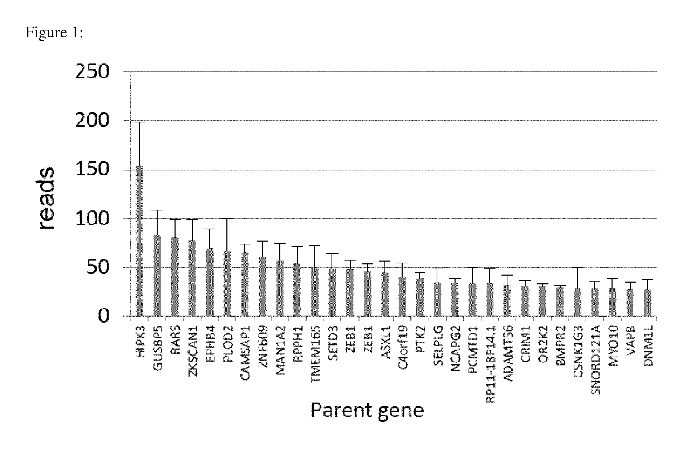

- **Figure 1:**: Expression of circRNA transcripts in human umbilical venous cord endothelial cells (HUVEC). Highest expressed circRNAs are shown.

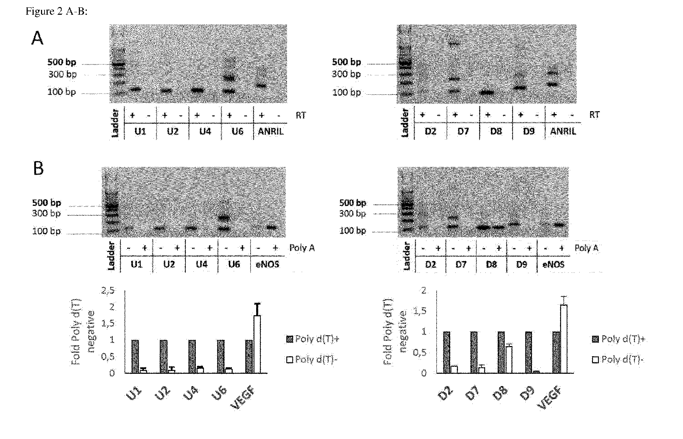

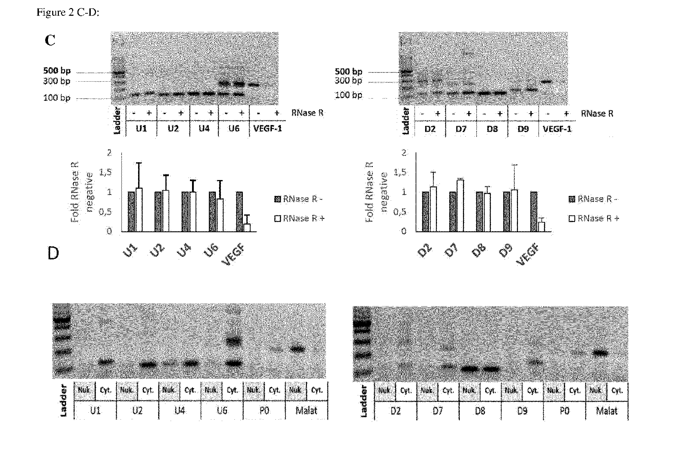

- **Figure 2:**: Biochemical characterization of selected circRNAs. A) Validation of circRNAs by RT-PCR in the presence or absence of reverse transcriptase (RT). ANRIL was used as control. B) RT-PCR of circRNAs in PolyA- or PolyA+ endothelial RNA. eNOS serves as control as a polyA+ mRNA. The lower panel shows statistical analysis of the data. C) RT-PCR after incubation of total RNA with or without RNase R. VEGF-1 serves as controls for a linear RNA. The lower panel shows statistical analysis of the data. D) RT-PCR of circRNAs in nuclear (Nuk.) and cytoplasmic (Cyt) fractions.

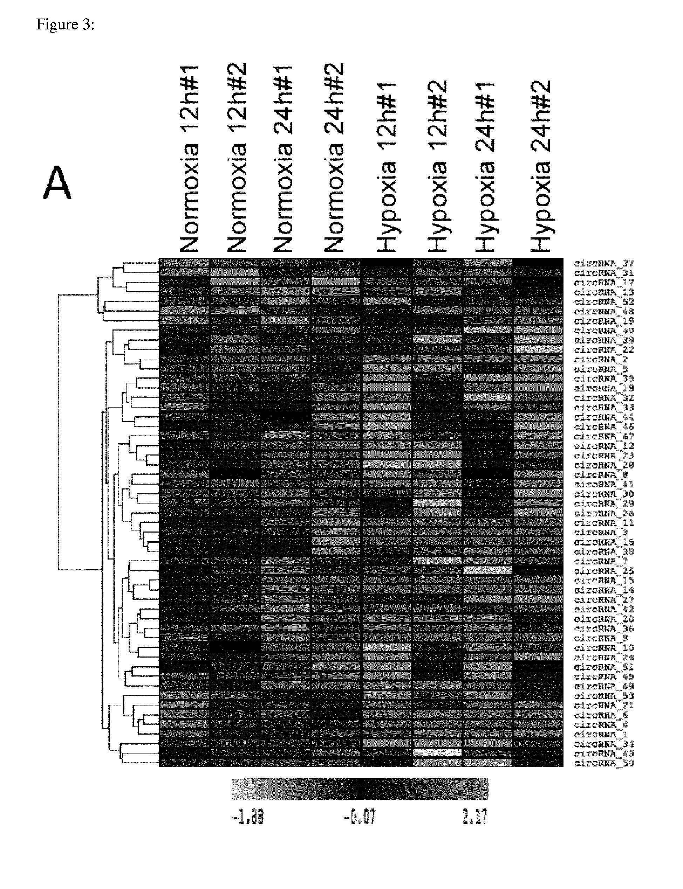

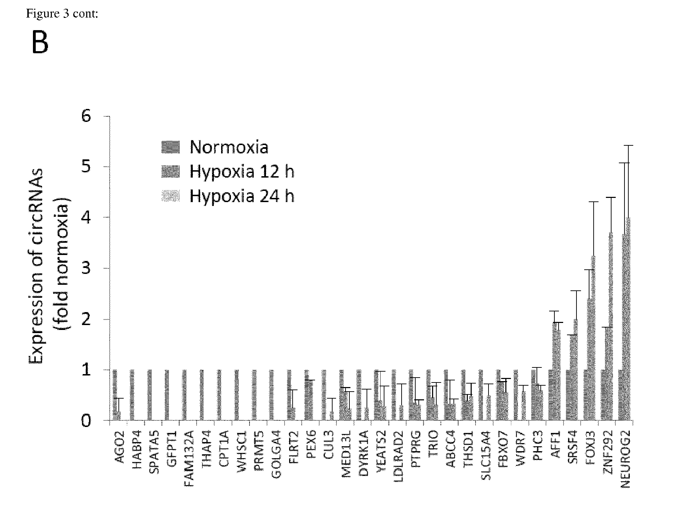

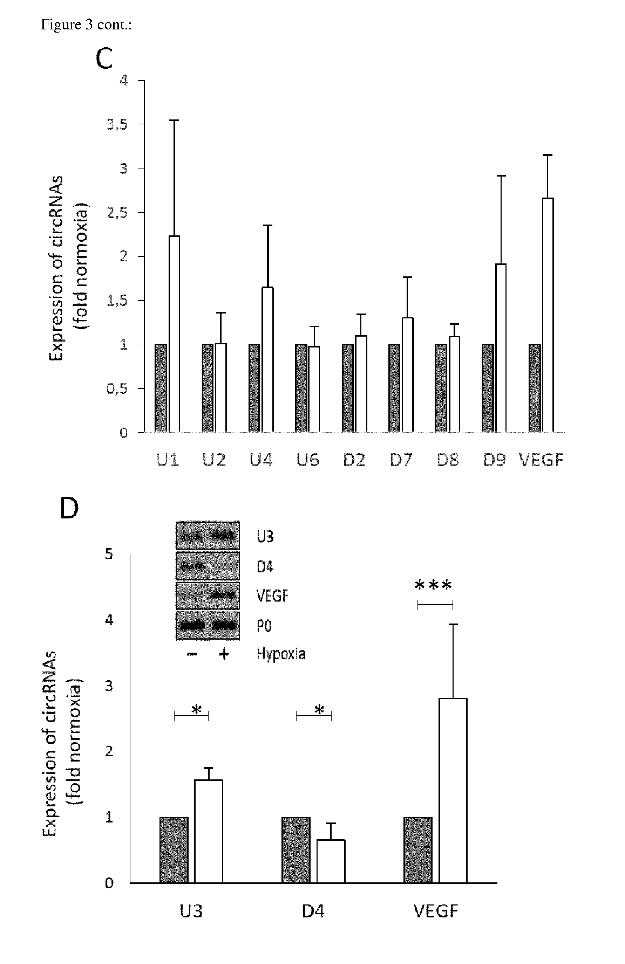

- **Figure 3:**: Characterisation of hypoxia-regulated circRNAs. A) Heat map showing hypoxia-regulated circRNAs in HUVEC (deep sequencing). B) Expression of significantly regulated circRNAs after 12 or 24 h of hypoxia. (Deep sequencing). C/D) Confirmation of hypoxia-regulated circRNAs by RT-PCR.

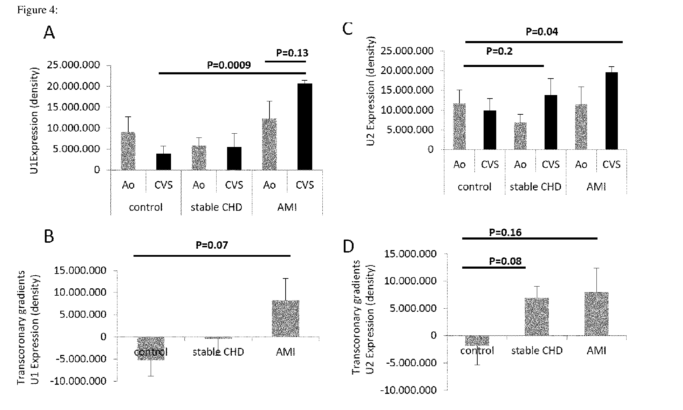

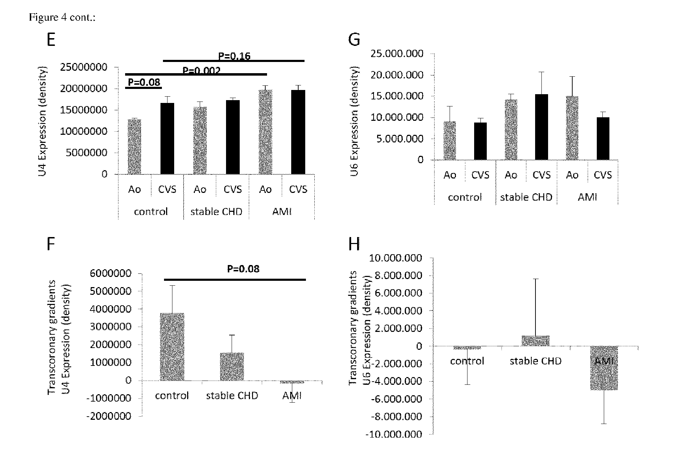

- **Figure 4:**: Regulation of circRNAs in patients with coronary artery disease. Expression was detected in plasma samples of patients without coronary artery disease (n=4), patients with stable coronary heart disease (CHD; n=4) and patients with acute myocardial infarction (AMI, n=4). Plasma was obtained from venous blood (coronary venous sinus (CVS)) or from the arterial blood (Aorta, Ao). Panel A, C, E, and G show expression of U1, U2, U4 and U6 in aortic and venous EDTA plasma. Panels B, D, F and H show the transcoronary gradients of U1, U2, U4 and U6, respectively.

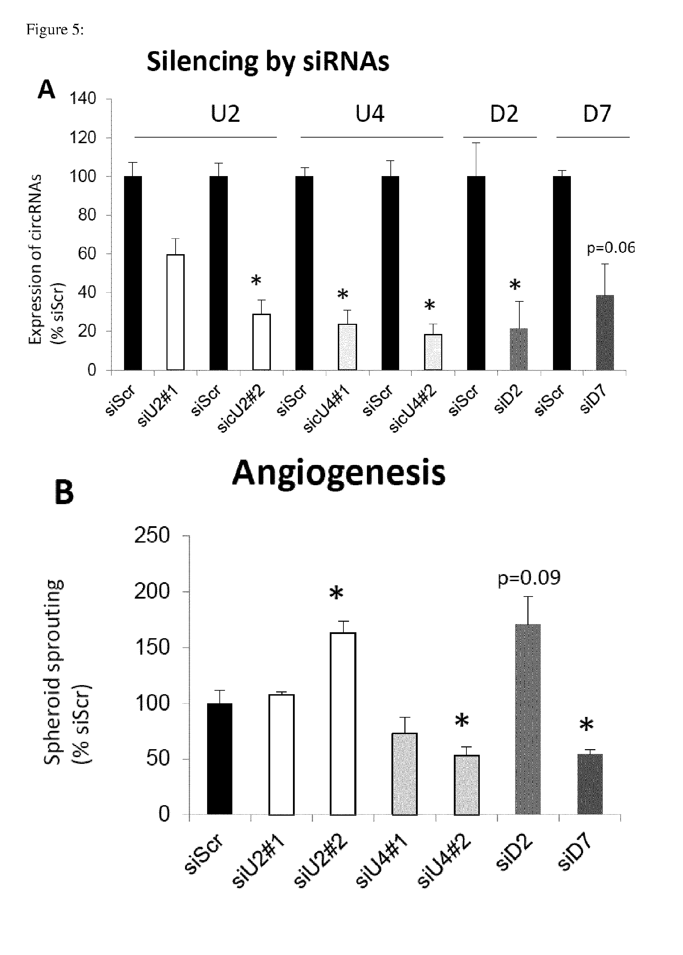

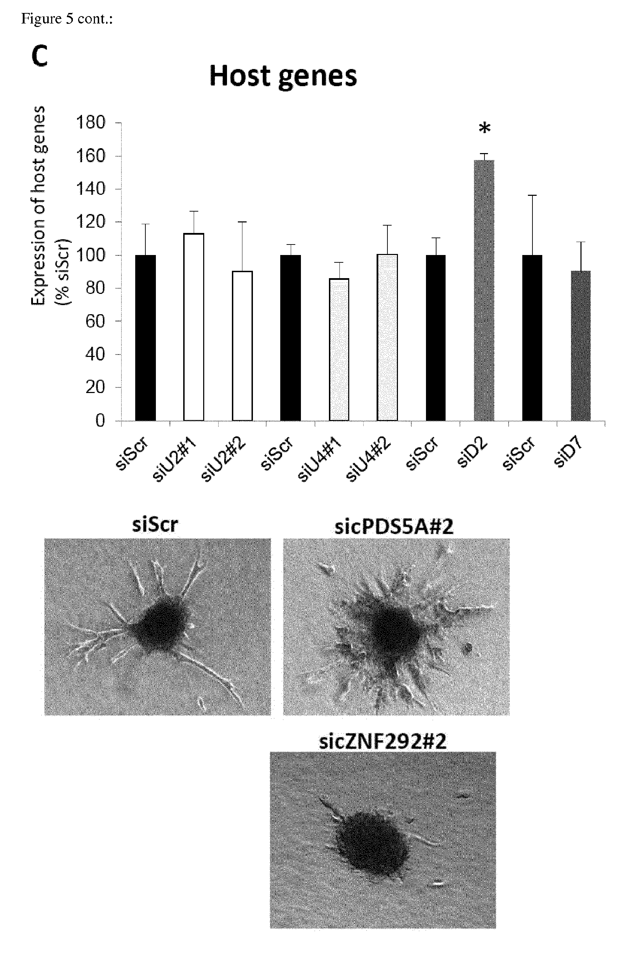

- **Figure 5:**: Angiogenic effects of selected circRNAs. CircRNAs were silenced with siRNAs directed against the newly generated back splicing site. Two sequences were used for silencing of U2 and U4 (indicated by #1 and #2). A) Expression of circRNAs after silencing with siRNAs. B) Spheroid spouting depicting the angiogenic activity of endothelial cells after silencing of circRNAs. Representative pictures are shown on in the right panel. C) mRNA expression of the linear parent genes after silencing of circRNAs. All data are n>3.

### EXAMPLES

### Material and Methods

### Cell culture

Endothelial cells were purchased from Lonza and were cultured under hypoxic (0.5% 02) or normoxic conditions in EMB medium plus supplements. RNA was isolated and 0.5 µg ribosomal RNA-depleted RNA was fragmented and primed for cDNA synthesis. The sequencing libraries were constructed using Illumina ® TruSeq™ RNA Sample Preparation Kits and were sequenced by Illumina HiSeq™ 2000 flowcell.

### Bioinformatic analysis

Computational analysis of deep sequencing data sets obtained from Ribo-minus RNA was performed according to Memczak et al.

### Detection of circRNAs

CircRNAs were detected by semiquantitative RT-PCR reaction after reverse transcription with MuLV Reverse Transcriptase (N808-0018, Invitrogen) using the KOD XtremeTM Hot-Start Polymerase Kit (71975-3, Novagen). Poly-A-RNA was isolated by means of polyd(T)25-Magnetic Beads (S14195, NEB). For RNase R digestion, RNA was incubated with 1 U RNase R (RNR-07250, Epicentre) at 37°C for 10 min followed by heat inactivation at 95°C for 3 min.

### Angiogenesis & EC survival

Functional studies were performed after transfection with siRNAs using 60 nM GeneTransII, and spheroid assays were performed as previously described.

### Example 1: Identification and characterization of endothelial circRNAs

Endothelial circRNAs were identified in ribosomal RNA-depleted RNA of HUVEC according to Memczak et al. The highest expressed transcripts in endothelial cells are shown in Figure 1.

Based on their basal expression levels and the identification of long reads that are covering the newly generated exon-exon or exon-intron boundaries, the inventors selected 16 circRNAs named U1-U7 (SEQ ID NO: 1 to 7; see table 1) and D1-D9 (SEQ ID NO 8 to 16; see table 1) and biochemically characterized U1, U2, U4, U6 and D2, D7, D8, D9. Reads of five selected circRNAs were derived from exon-exon back-splicing event, whereas two circRNAs show an exon-intron back-splicing and one derived from intron-intron head to tail joining (Table 1). To confirm the expression of these transcripts, the inventors performed RT-PCR experiments directed against the newly formed exon-exon, exon-intron or intron-intron boundaries and showed that all 8 selected candidates are expressed in HUVEC (Figure 2A) as well as in microvascular and aortic ECs.

Next, it was determined whether the selected transcripts are indeed circRNAs by testing their enrichment in poly-A-negative RNAs (as circRNAs are expected to have no Poly-A-tail) and resistance to 3'-5' exoribonuclease RNase R digestion (as circRNAs are expected to be resistant to exonuclease RNase R). Herein it is demonstrated that 7 out of 8 of the selected circRNAs are predominantly detected in the Poly-A-negative fraction (Figure 2B). In contrast, as expected, protein-coding mRNAs, such as endothelial NO-synthase (NOS3), were enriched in the poly-A-positive fraction (Figure 2B). Only D8 was also detected in poly-A+ RNA (Figure 2B). Furthermore, all eight transcripts were resistant to RNase R digestion (Figure 2C). As a control, the inventors used linear mRNA transcripts, such as VEGF-A, which was degraded (Figure 2C).

Moreover, the cellular localization of the 8 candidates was characterized and it is shown that all circRNAs except D8 are enriched in the cytoplasmic fraction (Figure 2D). In summary, seven out of the eight selected circRNAs fulfilled all the criteria for bona fide circRNAs.

### Example 2: Identification of hypoxia-regulated circRNAs

Since hypoxia is a key stimulus for angiogenesis, HUVEC were exposed to hypoxia and the expression of circRNAs was determined. Hypoxia induced a significant regulation of circRNAs as can be seen from heat map (Figure 3A) and the list of top regulated circRNAs (Figure 3B).

The regulation of 4 selected hypoxia-controlled circRNAs was validated by RT-PCR (Figure 3C/D) and we showed that U1, U3, and U4 are up-regulated whereas D4 expression was reduced by hypoxia. Interestingly, U1, U2 and U4 were increased in venous or aortic blood samples of patients with coronary artery disease (Figure 4). U2 was significantly increased in venous plasma in patients with acute myocardial infarction (AMI) (Figure 4C), whereas U1 and U4 were significantly increased in aortic plasma of patients with AMI (Figure 4A/E). U1 and U2 tend to be released by the heart in patients with stable coronary heart disease (CHD) or AMI as evidenced by positive transcoronary gradients (Figure 4B/D). U4 tend to be higher in transcoronary gradients of controls (Figure 4F). U6 did not show significant differences (Figure 4G/H)

### Example 3: circRNAs regulate angiogenic sprouting

One of the key questions is whether circRNAs have biological functions or are mere byproducts of splicing event. Therefore we used siRNAs directed against the sequences that are newly generated by back splicing to determine their biological functions in endothelial cells. siRNA-mediated silencing of U2 and D2 increased endothelial sprout formation, whereas silencing of U4 and D7 reduced spheroid sprouting (Figure 5A/B). Host genes were only regulated in case of D2 (Figure 5C) suggesting that the angiogenesis regulatory effect of U2, U4 and D7 is not related to an interference with splicing of the host gene.

The present invention confirms that circRNAs are also abundantly expressed in endothelial cells. We additionally characterized hypoxia controlled endothelial circRNAs and showed that highly expressed and significantly regulated circRNAs exhibits biological functions in endothelial cells. Selective depletion of circRNAs by siRNAs regulates angiogenic sprouting of endothelial cells suggesting that these circRNA exhibit angiogenic and endothelial-regulatory functions.

In addition, the present invention shows that hypoxia-regulated circRNAs can be measured in human plasma samples and U2 and U4 are increased in patients with acute myocardial infarction. Given that circRNAs are exonuclease resistance and exhibit a high stability, circRNAs may not only been detectable in tissue samples but may be used as circulating biomarkers for diseases.

**Table 2 circRNA sequences and backsplicing sites of the invention**

| **Name** | **Host gene** | **Back splice site)** | **circRNA sequence (5'-3') at back splice site. Back splice site underlined** | **SEQ ID NO:** | **Chromosome position (Back splice site GRCh37/hg19)** |
|---|---|---|---|---|---|
| | | **E= exon** | | | |
| | | **I= intron** | | | |
| U1 | DENN D4C | E_10/E_3 | AUGUUGUUUGCAUUGAC UUGGAUACGAACAUGUU AUAUGUAGUUCUAUAUG AAGGGAAAGAACGGCUU AUUCCAGGAUGU | 1 | Chr9: 19286766-19305525 |
| U2 | PDS5A | E_9/E_5 | CAACACAGAAUCGUCCU CUUUGGCAAUGUUUCU UGGACGAAUUUAGCUUG GGUUAAAUCAUAUAACA UCUGCUUUGAAU | 2 | Chr4: 39915230-39927553 |
| U3 | AFF1 | E_5/E_4 | CAAGCUCACCAAACUGA AGAUGCCUUCUCAGUCA GUUGAGUUUGUACAAUG ACGACAGAAACCUGCUU CGAAUUAGAGAG | 3 | Chr4: 87967317-87968746 |
| U4 | ZNF29 2 | E_4/I_1 | UGCACUAUUCUUUCCCA GGAACCAUUGGAUAAGG AUAAAGGCUUGCCCCAG AACACACACUAUAGAGC AGAUGCAGUAGC | 4 | Chr6: 87920168-87928449 |
| U5 | CUL3 | E_3/E_2 | AACAGCUAUGGUGAUGA UUAGAGACAUACUAAUG UACAUGAUGACCAUGGA UGAAAAAUAUGUAAACA GCAUUUGGACC | 5 | Chr2: 225400244-225422573 |
| U6 | MPP6 | E_10/E_3 | AUAGUAUUGAAUCCCAC UAGAUUUGGAACUACGG UGCCAUCAGCAAUGCAG CAAGUCUUGGAAAACCU UACGGAGCUGCC | 6 | Chr7:24663281-24708279 |
| U7 | HERC1 | E_27/E_22 | AGGAGGCAAGGAGAGA GUGGCCGAUUGCAUCAC UAUCAGAUAUCAGAAAA CCAGGACUCUGCAGAUG UUGAUCCUCAGG | 7 | Chr15: 63988322-64008672 |
| D1 | STT3B | E_3/E_2 | CAUUGCUAUUUUGCAC UUCAGUUCACAUACUAU UUAUGGGUUUAACUAUA GAUCAACACAUCAUCUU GCAUCUCAUGGG | 8 | Chr3: 31617887-31621588 |
| D2 | PTPR6 | I_1/I_1 | UCUCACCAAAGCCCUGG GGAAUGAAUGACUUGUU CAGGAGUGACAGAUUGC AGUACGUAUUUUAGGAC CUGCCACCCAUU | 9 | Chr3: 61608136-61609229 |
| D3 | SMAD 2 | E_6/E_2 | GAAACAAGUGACCAACA GUUGAAUCAAAGUAUGG ACACAGGUUCGAUACAA GAGGCUGUUUUCCUAGC GUGGCUUGCUGC | 10 | Chr18: 45391429-45423180 |
| D4 | THSD1 | E_3/E_3 | UCUGCAAAGGAGGAGUG CAUGCUAAUUCAGAGAA AUACAGUUCUUGGAGAA GCUGAAUAUCUUCUCUU GAGAGAGCCAGG | 11 | Chr13: 52971366-52972329 |
| D5 | TMEM 181 | E_6/E_4 | UGAACACCUGAAGCUCC CCAUCAAGGGAAUGAAC | 12 | Chr6: 159004985-159010814 |
| | | | UUCACACUAAAGCCAAU UCAAAUACUUUCAAAUC CACUGUCUACAU | | |
| D6 | PREX2 | E_7/E_4 | UUUUCGAUAAUCUUUUG GUGUACUGCAAAAGAAA ACACAGAAAGACAAGUU UCGUAUCUAUGAUGAAU AUUGUAGUAACC | 13 | Chr8: 68934270-68950527 |
| D7 | TRIO | E_11/E_9 | ACUGGACAUGUCAGUGU CCUUUCACACCCAUGUG AAAGAGGUCAGCCAAGA UGGGAAGUCGCUCCUUG ACAAGCUCCAGC | 14 | Chr5: 14316621-14336836 |
| D8 | MED1 3L | I_2/E_2 | UUUGUAGUAUUUAGAU UGGUAUCACAGUGCACC UGAAAAGCUGAACUCAC GGGAAUCAAAUGGCGUA GGUACAAUUUG | 15 | Chr12: 116668237-116675510 |
| D9 | ZMYN D8 | E_15/E_12 | AAAACCUCCACCACCGG CAGCAUCCUGAAUCUUA ACCUGGAUGAAUGAAAU CGAAGUAUGUCCAGAAU GUUAUCUAGCUG | 16 | Chr20: 45891031-45912392 |

**Table 3: extended circRNA sequences of the invention (cDNA sequence of the transcript: Uracil exchanged by Thymine)**

| **Name** | **Hostgene** | **SEQ ID** | **Sequence 5'-3'** |
|---|---|---|---|
| U1 | DENND4C | 17 | CTTTACCATTTATAGTTGGAGTTGACTCAAGGTATTTGATCTTC ATGACCCACCACAAGATGTTGTTTGCATTGACTTGGATACGAAC ATGTTATATGTAGTTCTATATGAAGGGAAAGAACGGCTTATTCC AGGATGTGAAGTGATCCTAGCCACACCCTATGGTCGCTGTGCCA ATGTCAACAATAGTTCAACTACT |
| U2 | PDS5A | 18 | ACTTGATCACTGAGAAGAGAGTTCTAAAAAGCTGAATAAAAAT TTCATTGCAATCTTCCAATTCAAAGCAGATGTTATATGATTTAA CCCAAGCTAAATTCGTCCAAGAAAACATTGCCAAAGAGGACGA TTCTGTGTTGCCAAATCAGAATCTTTGGAGCCAAACAATTTAGC TAGAAGTCGAACAACAGCTAATCGCT |
| U3 | AFF1 | 19 | GCCGGAGGACTATCGACAGCAGACCTTTGAAAAACAGACTTG AAAGTGCCTGCCAAAGCCAAGCTCACCAAACTGAAGATGCCTT CTCAGTCAGTTGAGTTTGTACAATGACGACAGAAACCTGCTTCG AATTAGAGAGAAGGAAAGACGCAACCAGGAAGCCCACCAAGA GAAAGAGGCATTTCCTGAAAAGATTCCC |
| U4 | ZNF292 | 20 | GAATTGCATTTTTAGCTACTCTAGCTCAAGAGACTGGGGTGTG GAAAAACCCGGTACTGTGCACTATTCTTTCCCAGGAACCATTGG ATAAGGATAAAGGCTTGCCCCAGAACACACACTATAGAGCAGA TGCAGTAGCCACAAATAACTTTGTGGGGATCTAGTAGCTCCCTT AAAGAATGAGGTGAGGCAGGCTGAA |
| U5 | CUL3 | 21 | CCTCAAAACTAAGACCACTGTTATTCTTACGCTGGATTTCTTGA ATTGCATTTTTCAGAAGGTCCCAAATGCTGTTTACATATTTTTCA TCCATGGTCATCATGTACATTAGTATGTCTCTAATCATCACCAT AGCTGTTTGATGATCATTCCAAGCTTGATTTAGCGTTTGAAGAA AGTTGTTATTCAATGAATTTAGT |
| U6 | MPP6 | 22 | AAAACATTAGTATTGATAGGAGCTCAAGGTGTAGGCCGAAGAA GCTTGAAAAACAGGTTCATAGTATTGAATCCCACTAGATTTGGA ACTACGGTGCCATCAGCAATGCAGCAAGTCTTGGAAAACCTTA CGGAGCTGCCCTCGTCTACTGGAGCAGAAGAAATAGACCTAAT TTTCCTCAAGGGAATTATGGAGAATCC |
| U7 | HERC1 | 23 | CTCTCTCAGCCTGTTCTTCCATTTCAGCTTCTTCATCAATAGTTC |
| | | | GAGTAAATGAATGCTCCTGAGGATCAACATCTGCAGAGTCCTG GTTTTCTGATATCTGATAGTGATGCAATCGGCCACTCTCTCCCTT GCCTCCTGTGTGTCCAACAGTGCCTAAACCAAAACACCCTGCTA GGAACTGCAGCCTCACAGACGTG |
| D1 | STT3B | 24 | TATTGCTATTGTACCAGGCTACATATCTCGGTCAGTAGCTGGAT CCTTTGATAATGAAGGCATTGCTATTTTGCACTTCAGTTCACAT ACTATTTATGGGTTTAACTATAGATCAACACATCATCTTGCATC TCATGGGTTCTATGAATTTTTAAATTGGTTTGATGAAAGAGCAT GGTATCCACTAGGAAGAATAGTA |
| D2 | PTPR6 | 25 | GTGCAGGGCTTTGATCATAGAGTGGATTTTCAAACATTGGAGCA GGAAGAGGCTCCCGACTCTCACCAAAGCCCTGGGGAATGAATG ACTTGTTCAGGAGTGACAGATTGCAGTACGTATTTTAGGACCTG CCACCCATTTTGATTTGAAAGATTATGTTGTCGGAATTTCAGAC ACTGCAGGTAAAGATCTAATTTTAC |
| D3 | SMAD2 | 26 | AGCAGTCTCTTCACAACTGGCGGCGTGAATGGCAAGATGGACG ACATGTTCTTACCAAAGGCAGCAAGCCACGCTAGGAAAACAGC CTCTTGTATCGAACCTGTGTCCATACTTTGATTCAACTGTTGGTC ACTTGTTTCTCCATCTTCACTGATATATCCAGGAGGTGGCGTTTC TACAAAAGTTTAAAACAAATCAAG |
| D4 | THSD1 | 27 | TTAGCACCATCAAAATACTGGAAATCCACATACACTGTGTCGTT GCTTAGTGCTACATGGCCTGGCTCTCTCAAGAGAAGATATTCAG CTTCTCCAAGAACTGTATTTCTCTGAATTAGCATGCACTCCTCCT TTGCAGAAAAATGGCTTCTGCTTGAAATGCCAAAGTCAAAGCA GTACTTATTCTTCCCCATGTCAAA |
| D5 | TMEM181 | 28 | GATTATTGTGGCTCACCTTGGCTACCTGAACTACACTCAGTATA CAGTGATAGTGGGATTTGAACACCTGAAGCTCCCCATCAAGGG AATGAACTTCACACTAAAGCCAATTCAAATACTTTCAAATCCAC TGTCTACATACAATCAGCAACTATGGCTGACATGTGTTGTTGAG TTGGATCAATCAAAAGGTATGGAGC |
| D6 | PREX2 | 29 | TAATGTGTGGAGTCTTACTGAAAATTTCTTCTGGAAATATTCAA GAACGGGTGTTTTTCTTTTCGATAATCTTTTGGTGTACTGCAAA AGAAAACACAGAAAGACAAGTTTCGTATCTATGATGAATATTG TAGTAACCATGAGAAGGCACAAAAATTACTTCTTGAACTCAAC AAAATAAGAACAATCCGGACATTTC |
| D7 | TRIO | 30 | CCATCAGCTGGAAGACCGGATTCAAGATTTCGTTCGGCGTGTTG AGCAGCGAAAGATCCTACTGGACATGTCAGTGTCCTTTCACACC CATGTGAAAGAGGTCAGCCAAGATGGGAAGTCGCTCCTTGACA AGCTCCAGCGGCCCTTGACTCCCGGCAGCTCCGATTCCCTGACA GCCTCTGCCAACTACTCCAAGGCCG |
| D8 | MED13L | 31 | ACAGAATTGGATCATCTTGGGCTGGGGCTGAAATTATGGGTCCA CAGTCCCCATGCCCTCCAAAATTGTACCTACGCCATTTGATTCC CGTGAGTTCAGCTTTTCAGGTGCACTGTGATACCAATCTAAAAT ACTACAAATCTACAATGCTGCTGCAAAATGGTATCATTCATGTT TTCAAAATCACCAGAATTCCATAC |
| D9 | ZMYND8 | 32 | AAGCAAAGTAAATCCAACTTACACAAGGCTCACAAAACCAGTT ATCTCGTTTTGGCAAGCAGCTAGATAACATTCTGGACATACTT CGATTTCATTCATCCAGGTTAAGATTCAGGATGCTGCCGGTGGT GGAGGTTTTGTCCGTTTTCGTCGTGATAGGAGCTGACAGTTGAG GAGAGAAGGGCTTTGGGCTGCCGGA |

### References

1. Thum T (2012) MicroRNA therapeutics in cardiovascular medicine. EMBO Mol Med 4: 3-14.
2. Uchida S, Dimmeler S (2014) Long non-coding RNAs in cardiovascular diseases. Circulation Research in press.
3. Biselli-Chicote PM, Oliveira AR, Pavarino EC, Goloni-Bertollo EM (2012) VEGF gene alternative splicing: pro- and anti-angiogenic isoforms in cancer. J Cancer Res Clin Oncol 138: 363-370.
4. Liu H, Tang L (2013) Mechano-regulation of alternative splicing. Curr Genomics 14: 49-55.
5. Memczak S, Jens M, Elefsinioti A, Torti F, Krueger J, et al. (2013) Circular RNAs are a large class of animal RNAs with regulatory potency. Nature 495: 333-338.
6. Jeck WR, Sorrentino JA, Wang K, Slevin MK, Burd CE, et al. (2013) Circular RNAs are abundant, conserved, and associated with ALU repeats. RNA 19: 141-157.
7. Rearick D, Prakash A, McSweeny A, Shepard SS, Fedorova L, et al. (2011) Critical association of ncRNA with introns. Nucleic Acids Res 39: 2357-2366.
8. Zhang Y, Zhang XO, Chen T, Xiang JF, Yin QF, et al. (2013) Circular intronic long noncoding RNAs. Mol Cell 51: 792-806.
9. Nigro JM, Cho KR, Fearon ER, Kern SE, Ruppert JM, et al. (1991) Scrambled exons. Cell 64: 607-613.
10. Capel B, Swain A, Nicolis S, Hacker A, Walter M, et al. (1993) Circular transcripts of the testis-determining gene Sry in adult mouse testis. Cell 73: 1019-1030.
11. Cocquerelle C, Mascrez B, Hetuin D, Bailleul B (1993) Mis-splicing yields circular RNA molecules. FASEB J 7: 155-160.
12. Surono A, Takeshima Y, Wibawa T, Ikezawa M, Nonaka I, et al. (1999) Circular dystrophin RNAs consisting of exons that were skipped by alternative splicing. Hum Mol Genet 8: 493-500.
13. Guo JU, Agarwal V, Guo H, Bartel DP (2014) Expanded identification and characterization of mammalian circular RNAs. Genome Biol 15: 409.
14. Burd CE, Jeck WR, Liu Y, Sanoff HK, Wang Z, et al. (2010) Expression of linear and novel circular forms of an INK4/ARF-associated non-coding RNA correlates with atherosclerosis risk. PLoS Genet 6: e1001233.
15. Hentze MW, Preiss T (2013) Circular RNAs: splicing's enigma variations. EMBO J 32: 923-925.
16. Hansen TB, Jensen TI, Clausen BH, Bramsen JB, Finsen B, et al. (2013) Natural RNA circles function as efficient microRNA sponges. Nature 495: 384-388.
17. Kaluza D, Kroll J, Gesierich S, Yao TP, Boon RA, et al. (2011) Class IIb HDAC6 regulates endothelial cell migration and angiogenesis by deacetylation of cortactin. EMBO J 30: 4142-4156.
18. Salzman J, Gawad C, Wang PL, Lacayo N, Brown PO (2012) Circular RNAs are the predominant transcript isoform from hundreds of human genes in diverse cell types. PLoS One 7: e30733.

## Claims

1. A nucleic acid, comprising
a. A sequence having at least 70% sequence identity to a circular RNA comprising a sequence shown in any of SEQ ID NO: 1 to 16,
b. A sequence which is complementary to or binds to a sequence having at least 70% sequence identity to a circular RNA comprising a sequence shown in any of SEQ ID NO: 1 to 16,
c. A sequence of at least 5 to 50 successive nucleic acid residues of a sequence having at least 70% sequence identity to a circular RNA comprising a sequence shown in any of SEQ ID NO: 1 to 16, and/or
d. A sequence which is complementary to or binds to a sequence of at least 5 to 50 successive nucleic acid residues of a sequence having at least 70% sequence identity to a circular RNA comprising a sequence shown in any of SEQ ID NO: 1 to 16.

2. The nucleic acid according to claim 1, which is a DNA or RNA, and which is single stranded or double stranded, and which is circular or linear.

3. An *in-vitro* or *ex-vivo* method of predicting, monitoring and/or diagnosing a cardiovascular disease in a subject, comprising the steps of:
a. Providing a biological sample from the subject,
b. Determining the level of one or more non-coding circular RNA transcripts (circRNA) in the biological sample, wherein the circRNA transcript is selected from a circRNA comprising a sequence shown in any of SEQ ID NO: 1 to 16;
c. Comparing the level of the circRNA in the biological sample to a control level of the one or more circRNA transcripts;
d. Predicting, monitoring and/or diagnosing the subject as having the cardiovascular disease when the level of the one or more circRNA in the biological sample is higher or lower compared to the control level.

4. The method according to claim 3, wherein the cardiovascular disease is selected from atherosclerosis, a coronary heart disease, an acute coronary symptom, preferably unstable angina pectoris or acute myocardial infarction (AMI), stable angina pectoris, stroke, preferably ischemic stroke, inflammation or autoimmune disease associated artheriosclerosis or restenosis.

5. The method according to claim 3 or 4, wherein step b. involves the use of at least one nucleic acid molecule according to claim 1 or 2, preferably wherein said nucleic acid molecule is a primer or a probe.

6. The method according to any of claims 3 to 5, wherein the biological sample is a venous or aortic plasma sample.

7. The method according to any of claims 3 to 6, wherein a higher level of a circRNA comprising the sequence shown in SEQ ID NO: 2 in a sample of venous plasma of the subject compared to the control level indicates acute myocardial infarction; and/or wherein a higher level of a circRNA comprising the sequence shown in SEQ ID NO: 1 and/or SEQ ID NO: 4 in a sample of aortic plasma of the subject compared to the control level indicates acute myocardial infarction.

8. A compound for use in the treatment of a disease associated with endothelial cell dysfunction, wherein the compound is an agonist of or is an antagonist of, a non-coding circular RNA (circRNA) comprising a sequence shown in any of SEQ ID NO: 1 to 16.

9. The compound for use according to claim 8, wherein the disease associated with endothelial cell dysfunction is pathological angiogenesis and wherein the compound is
a. An agonist of a circRNA comprising a sequence shown in SEQ ID NO: 2 and/or 9, or
b. An antagonist of a circRNA comprising a sequence shown in SEQ ID NO: 4 and/or 14;
Or wherein the disease associated with endothelial cell dysfunction is a cardiovascular disease and wherein the compound is
c. An antagonist of a circRNA comprising a sequence shown in SEQ ID NO: 2 and/or 9, or
d. An agonist of a circRNA comprising a sequence shown in SEQ ID NO: 4 and/or 14.

10. The compound according to claim 8 or 9, wherein the circRNA agonist is selected from a nucleic acid according to claim 1 or 2, or an circRNA expression construct comprising a nucleic acid according to claim 1 or 2, or a molecule that when contacted with a cell induces and/or increases the expression and/or function of a circRNA.

11. The compound according to any one of claims 8 to 9, wherein an antagonist of a circRNA is selected from an inhibitory nucleic acid, such as an antisense construct, for example an siRNA, dsRNA, LNA, GapmeR, or a molecule that represses the expression and/or function of a circRNA, preferably wherein the antagonist is an antisense construct comprising a sequence that is complementary to a circRNA sequence of a circRNA comprising a sequence shown in any of SEQ ID NO 1 to 16, or is complimentary to a sequence having at least 70% sequence identity to a circRNA sequence of a circRNA comprising a sequence shown in any of SEQ ID NO: 1 to 16.

12. An *in-vitro* or *ex-vivo* method for modulating endothelial cell function comprising contacting an endothelial cell with an agonist or antagonist of a circRNA, wherein the circRNA comprises a sequence shown in any of SEQ ID NO: 1 to 16.

13. An *in-vitro* or *ex-vivo* method for the generation of vascular tissue material, the method comprising the steps of contacting an endothelial cell with an antagonist of a circRNA comprising a sequence shown in SEQ ID NO: 2 and/or 9, or an agonist of a circRNA comprising a sequence shown in SEQ ID NO: 4 and/or 14, preferably wherein the vascular tissue material is a blood vessel.

14. A pharmaceutical composition, comprising an agonist or antagonist of a circRNA comprising a sequence shown in any of SEQ ID NO: 1 to 16, preferably the pharmaceutical composition comprising an isolated nucleic acid according to claim 1 or 2, and optionally further comprising a pharmaceutically acceptable carrier and/or excipient.

15. An *in-vitro or ex-vivo* use of an agonist or antagonist of a circRNA comprising a sequence shown in any of SEQ ID NO: 1 to 16, in the modulation of endothelial cell function.
